# DEMANDE DE BREVET EUROPEEN

(11) **EP 0 633 243 A1**
(43) Date de publication de la demande: **11.01.1995**
(21) Numéro de dépôt: 94401526.2
(22) Date de dépôt: 04.07.1994
(51) Int. Cl.: C07C 227/18, C07C 229/12, C07C 229/22, C07C 229/36, C07D 207/16, C07D 233/64, A61K 7/50, C11D 1/10, C11D 1/18, C11D 1/34

(54) **Procédé de préparation d'un composé hydroxylé d'amine secondaire ou tertiaire**

(30) Priorité: 06.07.1993 FR 9308245
(71) Demandeur: CHEMOXAL S.A., F-75321 Paris 07 (FR)
(72) Inventeur: Hamon, Catherine, F-92320 Chatillon sous Bagneux (FR); Boiteux, Jean-Pierre, F-80710 saix (FR); Teral, Gilles, F-75013 Paris (FR); Ledon, Henry, F-78000 Versailles (FR)
(74) Mandataire: Caen, Thierry Alain

(57) **Abrégé**

Un procédé de préparation d'un composé hydroxylé d'amine secondaire ou tertiaire présentant une fonction hydroxyle en β de la fonction amine, selon lequel on fait réagir un composé amine primaire ou secondaire avec un composé comportant une fonction époxy en présence d'un catalyseur de transfert de phase.

## Description

La présente invention concerne un nouveau procédé de préparation d'un composé hydroxylé d'amine secondaire ou tertiaire à partir d'un composé amine respectivement, primaire ou secondaire ne nécessitant pas la mise en oeuvre de solvant organique tel les alcools ou les cétones.

Il est connu de préparer des composés hydroxylés d'amine secondaire ou tertiaire, comportant une fonction hydroxyle en β de la fonction amine, en faisant réagir un composé amine, respectivement, primaire ou secondaire, avec un composé comportant une fonction époxy, en milieu solvant organique, constitué généralement par un alcool ou une cétone.

Les composés ainsi formés présentent l'inconvénient de comporter des traces de solvant organique de procédé, pouvant les rendre impropres à une utilisation en cosmétique ou en pharmacie ou des produits exempts de solvant organique sont souvent requis.

Le brevet français 2.099.030 décrit un procédé de préparation d'un composé N -(hydroxyhydrocarbyl) - N-(alkylaminocarboxylates) par réaction entre un composé 1, 2-époxyde d'hydrocarbure et un acide N-substitué aminocarboxylique, dans un milieu aqueux, en présence ou non d'un alcool.

Cependant, en vue de l'obtention de rendements acceptables, cette réaction doit être réalisée dans un réacteur sous très forte agitation, incompatible avec une mise en oeuvre à l'échelle industrielle.

De plus, le brevet français 2.099.030 préconise de purifier le produit issu de la réaction en le traitant au moyen d'un solvant organique porté à reflux. On comprend que cette méthode de purification conduit à l'obtention d'un produit ne pouvant être exempt de solvant organique, et que par ailleurs, la mise en oeuvre d'un solvant porté à reflux présente des risques importants pour la sécurité des personnes.

Par ailleurs, H. Rutzen dans Fette, Seifen, Anstrichmittel, vol.84, n° 3, 1982, Hamburg, pages 87-92, "Quaterernierung von tertiären Aminsalzen mit langkettigen Epoxiden" décrit un procédé de préparation de composés ammonium quaternaire par réaction d'un composé amine tertiaire avec un composé comportant une fonction époxy, en présence d'un catalyseur de transfert de phase.

La présente invention a pour principal objet un procédé de préparation d'un composé, hydroxylé d'amine secondaire ou tertiaire, pouvant être mise en oeuvre en l'absence de tout solvant organique et, pouvant être effectué avec des vitesses d'agitation compatibles avec les réacteurs industriels existants.

L'invention concerne ainsi un procédé de préparation d'un composé hydroxylé d'amine secondaire ou tertiaire présentant une fonction hydroxylé en β de la fonction amine à partir d'un composé amine, respectivement, primaire ou secondaire, caractérisé en ce que on fait réagir ledit composé amine primaire ou secondaire avec un composé comportant une fonction époxy en présence (i) d'un catalyseur de transfert de phase et (ii) d'une base minérale, puis, le cas échéant, on sépare ledit composé hydroxylé d'amine secondaire ou tertiaire formé dudit catalyseur de transfert de phase.

Il a été en effet constaté que la présence d'un catalyseur de transfert de phase dans le milieu réactionnel permettait de résoudre les problèmes exposés ci-dessus. Toutefois, il est indispensable d'effectuer la réaction en présence d'une base minérale, tout au moins quand l'amine primaire ou secondaire est sous forme libre, cela en vue de l'obtention d'un rendement convenable.

Dans le cadre de la présente invention, un catalyseur de transfert de phase est un composé chimique permettant la réaction entre deux corps chimiques se trouvant dans des phases différentes, tels par exemple, deux corps liquides de nature différente.

Avantageusement, le composé d'amine primaire ou secondaire comporte au moins un groupement oxygéné salifiable tel qu'un groupement -COO⁻, -SO₃⁻, -PO₄H⁻, -SO₄⁻ ou -PO₃²⁻, ledit groupement étant sous forme libre ou salifié. On entend ici par forme libre le fait que le groupement oxygéné salifiable présente un contre-ion H⁺, et par forme salifiée, le fait que le contre-ion consiste en un cation organique ou minéral, tel NH₄⁺ ou un cation d'un métal alcalin ou alcalino-terreux, tels le sodium, le potassium ou le calcium.

Plus particulièrement, le composé amine primaire ou secondaire peut consister en un composé carboxyaminé comportant 1 ou 2 groupes carboxyliques à l'état libre ou salifié.

De tels composés carboxyaminés peuvent notamment consister en des acides aminés, des oligopeptides, des peptides, des acides aminés N-acylés des oligopeptides N-acylés, des composés carboxyaminés hétérocycliques mono- ou polycycliques comportant de 3 à 14 atomes de carbone et au moins un atome d'azote en tant qu'hétéroatome, ou des mélanges de ces composés carboxyaminés.

Lesdits acides aminés peuvent consister en des acides a aminés tels que la sarcosine, l'asparagine, la valine, la leucine, l'isoleucine, la thréonine, la méthionine, la phénylalanine, le tryptophane, la lysine, l'hydroxylysine, l'alaline, l'arginine, l'acide aspartique, la cystine, la cystéine, l'acide glutamique, la glycine, l'histidine, la proline, l'hydroxyproline, la sérine, la tyrosine, la glutamine, ou des mélanges de ces acides aminés.

Ces acides aminés peuvent se présenter sous la forme D, ou, de préférence, sous la forme L ou la forme D,L.

Les acides aminés N-acylés et les oligopeptides N-acylés mentionnés ci-dessus sont bien connus. Ils peuvent notamment être préparés selon les procédés décrits dans la demande de brevet FR 2.619.711 ou la demande de brevet JP-03/294298.

Les composés carboxyaminés hétérocycliques pouvant être mis en oeuvre dans le procédé de l'invention peuvent comporter, outre un hétéroatome d'azote, 1 à 3 autres hétéroatomes choisis parmi N, O et S. Des composés de ce type peuvent consister en les dérivés carboxyliques de la morpholine, de la pyridine, de la pyrazine, de la pyrimidine, de la purine, de la quinoléine, et de l'isoquinoléine, de l'imidazole, du pyrazole, du thiazole, ou de l'oxazole ou un composé tel l'acide nicotinique. Un composé amine primaire ou secondaire comprenant un groupement -SO₃⁻ peut consister en la taurine ou la méthyl-taurine.

En tant qu'amine primaire ou secondaire pouvant être utilisé comme produit de départ dans le procédé selon la présente invention, on peut également citer des composés tels la chitine ou ceux de formule (I) :

R - NH - G (I)

dans laquelle R est l'hydrogène ou un radical en C₁ - C₃₀ et G est le reste d'un monosaccharide, d'un disaccharide, d'un polysaccharide ou d'un hexitol.

De préférence R est l'hydrogène ou un radical alkyle en C₁ - C₄.

G peut être un reste d'un monosaccharide tel le galactose, le fructose, le glucose, le mannose, le xylose, l'arabinose, le lyxose, le ribose ou le ribulose d'un disaccharide tel le saccharose, le maltose ou le lactose, d'un polysaccharide telle la cellulose ou l'amidon ou d'un hexitol, tel le sorbitol, le mannitol, ou le galactitol.

Les composés de formule (I) préférés consistent en la glucamine, la N-méthylglucamine et la glucosamine.

Bien entendu, on peut mettre en oeuvre un des composés amine primaire ou secondaire décrits ci-dessus, ou des mélanges de ceux-ci.

Un composé comportant une fonction époxy pouvant être mis en oeuvre dans le procédé selon l'invention peut consister en un composé de formule (II) 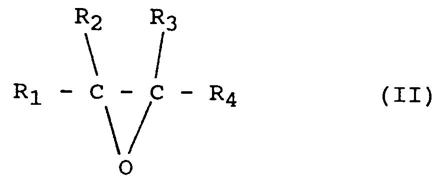
dans laquelle les substituants R₁, R₂, R₃, et R₄, identiques ou différents, représentent :
(i) l'hydrogène
(ii) un groupe R₅ [(CH₂)ₛX]ₜ, R₅ étant un radical aliphatique, linéaire ou ramifié, saturé ou insaturé comportant de 1 à 30 atomes de carbone, un radical cycloaliphatique comportant de 3 à 12 atomes de carbone ou un radical aryle, R₅ étant ou non substitué par une à trois fonctions hydroxyles ou amines, ou
(iii) un groupe R₆COOR₇, où R₆ est un radical alkylène en C₁ - C₁₀ ou R₆ représente une liaison covalente et R₇ est H ou un radical aliphatique, saturé ou insaturé, linéaire ou ramifié, comportant de 1 à 30 atomes de carbone, de préférence de 1 à 4 atomes de carbone,
   X est l'hydrogène ou le soufre, s est égal à 0 ou est compris entre 1 et 4 et t est égal à 0 ou est compris entre 1 et 20, sous réserve que l'un au moins de R₁, R₂, R₃ et R₄ est différent de l'hydrogène.

Avantageusement trois des substituants R₁, R₂, R₃ et R₄ représentent l'hydrogène, le quatrième de ces substituants étant différent de l'hydrogène ; plus particulièrement ce dernier substituant peut être un radical alkyle en C₁ - C₂₀, de préférence en C₈ - C₁₄.

Les catalyseurs de transfert de phase mis en oeuvre peuvent être ceux décrits dans W.P. Weber, G. W. Gorel "Phase Transfer Catalysis in Organic Synthesis" - Springer-Verlag, Berlin, Heidelberg, New-York (1977) ; C.M. STARKS, C. LIOTTA" Phase Transfer Catalysis. Principles and Techniques" - Academic Press, New York, San Francisco, London (1978) ; ou E.V. DEHMLOW, E.S. DEHMLOW " Phase Transfer Catalysis" - VCH Verlagsgesellschaft, Weinheim (1980 et 1983). De préférence, le catalyseur de transfert de phase est un composé d'ammonium quaternaire, tel un halogénure d'un composé d'ammonium quaternaire, comme les bromures et chlorures de tétralkylammonium ou de trialkylaryl-ammonium. De tels composés peuvent plus particulièrement consister en le bromure de didodécyldiméthylammonium, le chlorure de trioctylméthylammonium ou le bromure de dodécyldiméthylbenzylammonium. Le catalyseur de transfert de phase peut encore être un dérivé organique de l'ion phosphonium, comme les halogénures de tétraalkyle phosphonium, de tétraryle phosphonium ou de triarylalkyle phosphonium. De tels composés peuvent notamment consister en les bromures ou chlorures de tétrabutyle phosphonium, de tétraphényle phosphonium, de triphénylméthyle phosphonium ou de tétrabutyle phosphonium.

La réaction entre le composé amine primaire ou secondaire et le composé comportant une fonction époxy peut être réalisée en présence ou non d'un solvant, lequel solvant consiste de préférence en l'eau, par exemple l'eau dans laquelle peut être dissous ledit composé amine primaire ou secondaire. La concentration en composé amine primaire ou secondaire dans le milieu réactionnel peut être comprise entre 0,5 et 60 moles/litre, de préférence entre 1 et 20 moles/litre.

En cours de réaction, on peut éliminer en continu l'eau formée.

Avantageusement, la réaction est réalisée en présence d'une base minérale choisie parmi LiOH, Na₂CO₃, NaHCO₃, KHCO₃, K₂CO₃, NH₄OH, ou, de préférence, NaOH ou KOH.

Le rapport molaire entre le composé amine primaire ou amine secondaire et la base est compris habituellement entre 0,5 et 5 et, de préférence, il est de l'ordre de 1.

Le rapport molaire entre le composé comportant une fonction époxy et le composé amine primaire ou secondaire peut être compris entre 0,5 et 10, et de préférence entre 0,8 et 1,20.

La teneur en poids du catalyseur de transfert de phase mis en oeuvre est généralement comprise entre 0,1 et 10 % du poids du composé amine primaire ou secondaire.

La réaction entre le composé comportant une fonction époxy et le composé amine primaire ou secondaire peut être effectuée à une température supérieure à 90°C, de préférence à une température comprise entre 95°C et 160°C, plus préférentiellement comprise entre 95 et 125°C. Cette réaction est généralement réalisée à la pression atmosphérique ou à une pression voisine de celle-ci. Une vitesse d'agitation du milieu réactionnel inférieure à 1000 tours/minute, de préférence comprise entre 500 et 50 tours/minute, est généralement mise en oeuvre.

A la fin de la réaction entre le composé amine primaire ou secondaire et le composé comportant une fonction époxy, on obtient un composé hydroxylé d'amine secondaire ou tertiaire présentant une fonction hydroxyle en béta de la fonction amine en mélange avec le catalyseur de transfert de phase. Si l'on désire obtenir uniquement ledit composé hydroxylé, on peut éliminer le catalyseur de transfert de phase selon des méthodes classiques, bien connues de l'homme du métier.

Le composé hydroxylé d'amine secondaire ou tertiaire obtenu peut être
un composé de formule (III) : 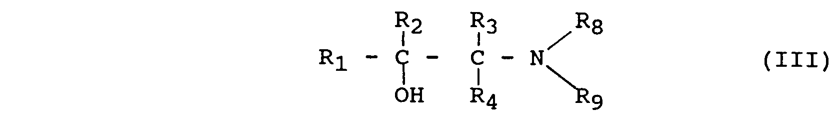
un composé de formule (IV) 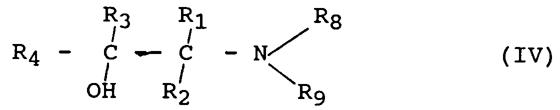
ou un mélange de composés de formules (III) et (IV).

Dans les formules (III) et (IV), R₁, R₂, R₃ et R₄, identiques ou différents, sont tels que définis ci-dessus, sous réserve que dans la formule (III), l'un de R₁ et R₂ est différent de l'hydrogène et que dans la formule (IV), l'un de R₃ et R₄ est différent de l'hydrogène,
R₈ et R_{9,}, identiques ou différents, représentent :
· l'une des significations de R₁
· un groupe R₁₀ - ZM, R₁₀ étant un radical aliphatique, linéaire ou ramifié, saturé ou insaturé ou un radical aryle, Z est un groupement oxygèné salifiable tel que -COO⁻, -SO₃⁻ ou -PO₃²⁻, -SO₄⁻ ou HPO₄⁻ et M un contre-ion, tel *NH₄* ou un cation d'un métal alcalin ou alcalino-terreux,
· un résidu d'un monosaccharide, d'un disaccharide, d'un polysaccharide ou d'un hexitol tels ceux définis plus haut,
· un groupe 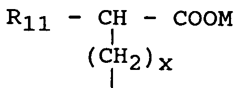 où R₁₁ est un reste d'un acide aminé, d'un oligopeptide ou d'un peptide, M a la signification indiquée ci-dessus et x est O ou un entier compris entre 1 et 5, ou
· R₈ et R₉ ensemble avec l'atome d'azote auxquels ils sont liés, forment un hétérocycle monocyclique ou polycyclique comportant de 3 à 14 atomes de carbone et, le cas échéant, 1 à 3 hétéroatomes additionnels choisis parmi 0, S et N,

ledit hétérocycle étant substitué ou non par 1 ou 3 fonctions carboxyliques et/ou hydroxyles.

Selon un autre aspect, l'invention concerne une composition comportant au moins un composé hydroxylé d'amine secondaire ou tertiaire présentant une fonction hydroxylée en béta de la fonction amine et un catalyseur de transfert de phase, ladite composition étant substantiellement exempte de solvant organique tels les alcools et les cétones.

Un composé hydroxylé préféré peut consister en un composé de formule (III), un composé de formule (IV) tels que définis plus haut, ou un mélange de ceux-ci. Avantageusement, le catalyseur de transfert de phase compris dans la composition est un dérivé organique de l'ion phosphonium ou un composé ammonium quaternaire et, de préférence, un halogénure d'un composé ammonium quaternaire, tels ceux définis ci-dessus.

Le rapport pondéral entre le catalyseur de transfert de phase et ledit composé hydroxylé présents dans ladite composition selon l'invention, peut être compris entre 0,001 et 0,1, de préférence entre 0,001 et 0,05.

Une telle composition peut être préparée selon le procédé de l'invention décrit ci-dessus, l'étape de séparation entre ledit composé hydroxylé d'amine secondaire ou tertiaire n'étant, bien entendu, pas réalisée.

Dans la mesure où la composition selon l'invention, telle que définie ci-dessus, est exempte de solvant organique, elle peut être avantageusement utilisée comme agent tensio-actif, notamment dans une composition cosmétique, pharmaceutique, hygiénique ou alimentaire. Une telle composition peut être également utilisée en tant qu'agent détergent.

Les exemples qui suivent ont pour but d'illustrer la présente invention.

Dans ces exemples, on a utilisé en tant que catalyseur de transfert de phase de l'Amonyl® Br 1244 qui est un bromure de dodecyldiméthylbenzylammonium, et de l'Aliquat® 336 qui est un chlorure de trioctylméthylammonium.

### Exemple 1

Dans un réacteur de 100 ml surmonté d'un Dean Stark, lui-même surmonté d'un réfrigérant et caractérisé par une agitation mécanique à l'aide d'une pale de type ancre (500 tours/min), on introduit 3,08 g de potasse en pastille (55 mmoles) et 10 ml d'eau distillée. On refroidit le mélange réactionnel à 5°C et on ajoute par fraction 4,90g de sarcosine (55 mmoles). On agite pendant 15 min. en laissant la température remonter jusqu'à 20°C. On ajoute 0,3 g d'Amonyl® Br1244 (6% masse/masse de Sarcosine) et on agite à nouveau à 500 tours/min durant 15 min à 20°C. 10,0 g d'époxydodécane (55 mmoles) sont additionnés. Le brut réactionnel est chauffé à 100°C durant 8 heures et refroidi à 20°C.

Le N-(2'-hydroxydodécyl) sarcosinate de potassium est obtenu avec 86 % de rendement.

A titre comparatif, on a reproduit l'essai ci-dessus sans catalyseur de transfert de phase. Le produit attendu n'a pu être synthétisé qu'avec un rendement de 74 %.

A titre comparatif, on a encore reproduit l'essai ci-dessus, mais cette fois en l'absence de potasse. Le produit de réaction détecté est obtenu avec un rendement inférieur à 7%.

### Exemple 2

Dans un réacteur identique à celui de l'exemple 1, on introduit sous agitation 3,08 g de potasse en pastilles (55 mmoles) et 10 ml d'eau distillée. On refroidit le mélange réactionnel à 5°C et on ajoute par fraction 4,90 g de sarcosine (55 mmoles). On agite pendant 15 min. en laissant la température remonter jusqu'à 20°C. On ajoute 0,02g d'Aliquat® 336 (0,4 % masse/masse de Sarcosine) et on agite à nouveau à 500 trs/min durant 15 min. à 20°C. 10,0g d'époxydodécane (55 mmoles) sont additionnés. Le brut réactionnel est chauffé à 100°C durant 8 heures et refroidi à 20°C.

Le N-(2'-hydroxydodécyl) sarcosinate de potassium est obtenu avec 87 % de rendement.

### Exemple 3

Dans un réacteur identique à celui de l'exemple 1, on introduit sous agitation 456 mg de soude en pastilles (11 mmoles) et lml d'eau distillée. On refroidit le mélange réactionnel à 5°C et on ajoute par fraction 1,02 g de Sarcosine (11 mmoles). On agite pendant 15 min. en laissant la température remonter jusqu'à 20°C. On ajoute 51mg d'Aliquat® 336 (0,5 % masse/masse de Sarcosine) et on agite à nouveau à 500 tours/min durant 15 min à 20°C. 2,10 g d'époxydodécane (11 mmoles) sont additionnés. Le brut réactionnel est chauffé à 100°C durant 8 heures et refroidi à 20°C.

Le N-(2'-hydroxydodécyl) sarcosinate de sodium est obtenu avec 85 % de rendement.

### Exemple 4

Dans un réacteur identique à celui de l'exemple 1, on introduit sous agitation 3,08 g de potasse en pastilles (55 mmoles) et lml d'eau distillée. On refroidit le mélange réactionnel à 5°C et on ajoute par fraction 4,90 g de Sarcosine (55 mmoles). On agite pendant 15 min en laissant la température remonter jusqu'à 20°C. On ajoute 0,3 g d'Aliquat® 336 (6 % masse/masse de Sarcosine) et on agite à nouveau à 500 tours/min durant 15 min à 20°C. 10,0 g d'époxydodécane (55 mmoles) sont additionnés. Le brut réactionnel est chauffé à 100°C durant 8 heures et refroidi à 20°C.

Le N-(2'-hydroxydodécyl) sarcosinate de potassium est obtenu avec 97 % de rendement.

A titre comparatif, on a répété l'essai ci-dessus, sans catalyseur de transfert de phase. Un rendement de 78 % a été obtenu.

### Exemple 5

Dans un réacteur identique à celui de l'exemple 1, on introduit sous agitation 3,08 g de potasse en pastilles (55 mmoles) et 10ml d'eau distillée. On refroidit le mélange réactionnel à 5°C et on ajoute par fraction 6,40g de proline (55 mmoles). On agite pendant 15 min en laissant la température remonter jusqu'à 20°C. On ajoute 0,3 g d' Aliquat® 336 (6 % masse/masse de Proline) et on agite à nouveau à 500 tours/min durant 15 min à 20°C. 10,0 g d'époxydodécane (55 mmoles) sont additionnés. Le brut réactionnel est chauffé à 100°C durant 8 heures et refroidi à 20°C.

Le N-(2'-hydroxydodécyl) prolinate de potassium est obtenu avec 98 % de rendement.

### Exemple 6

Dans un réacteur identique à celui de l'exemple 1, on introduit sous agitation 3,08 g de potasse en pastilles (55 mmoles) et 10ml d'eau distillée. On refroidit le mélange réactionnel à 5°C et on ajoute par fraction 6,40 g de D,L-Thréonine (55 mmoles). On agite pendant 15 min en laissant la température remonter jusqu'à 20°C. On ajoute 0,3 g d'Aliquat® 336 (5 % masse/masse de D,L-Thréonine) et on agite à nouveau à 500 tours/min durant 15 min. à 20°C. 10,0 g d'époxydodécane (55 mmoles) sont additionnés. Le brut réactionnel est chauffé à 100°C durant 8 heures et refroidi à 20°C.

Le N-(2'-hydroxydodécyl) D,L-thréoninate de potassium est obtenu avec 81 % de rendement.

A titre comparatif, on a reproduit cet essai sans catalyseur de transfert de phase. Aucun produit de condensation n'est formé. Deux phases limpides sont observées, l'une contenant l'époxyalcane, l'autre l'acide aminé.

A titre comparatif, on a encore reproduit l'essai ci-dessus, mais cette fois sans adjonction de potasse. Aucun produit de réaction n'a été détecté.

### Exemple 7

Dans un réacteur identique à celui de l'exemple 1, on introduit sous agitation 3,08 g de potasse en pastilles (55 mmoles) et 10 ml d'eau distillée. On refroidit le mélange réactionnel à 5°C et on ajoute par fraction 8,50g de L-Histidine (55 mmoles). On agite pendant 15 min en laissant la température remonter jusqu'à 20°C. On ajoute 0,3 g d'Aliquat® 336 (3,5% masse/masse de L-Histidine) et on agite à nouveau à 500 tours/min durant 15 min à 20°C. 10,0 g d'époxydodécane (55 mmoles) sont additionnés. Le brut réactionnel est chauffé à 100°C durant 8 heures et refroidi à 20°C.

Le N-(2'-hydroxydodécyl)L-Histidinate de potassium est obtenu avec un rendement de 62 %.

A titre comparatif, on a reproduit cet essai sans catalyseur de transfert de phase. Aucun produit de condensation n'est formé. Deux phases limpides sont observées, l'une contenant l'époxyalcane, l'autre l'acide aminé.

### Exemple 8

Dans un réacteur identique à celui de l'exemple 1, on introduit sous agitation 3,03 g de potasse en pastilles (54 mmoles) et 10 ml d'eau distillée. On refroidit le mélange réactionnel à 5°C et on ajoute par fraction 7,15 g de L-Asparagine (54 mmoles). On agite pendant 15 min en laissant la température remonter jusqu'à 20°C. On ajoute 0,3 g d'Aliquat® 336 (4% masse/masse de L-Asparagine) et on agite à nouveau à 500 tours/min durant 15 min à 20°C. 9,82 g d'époxydodécane (54 mmoles) sont additionnés. Le brut réactionnel est chauffé à 100°C durant 8 heures, refroidi à 20°C.

Le N-(2'-hydroxydodécyl) L-Asparaginate de potassium est obtenu avec un rendement de 64 %.

A titre comparatif, on a reproduit cet essai sans catalyseur de transfert de phase. Aucun produit de condensation n'est formé. Deux phases limpides sont observées, l'une contenant l'époxyalcane, l'autre l'acide aminé.

### Exemple 9

Dans un réacteur identique à celui de l'exemple 1, on introduit sous agitation 1,88 g de potasse en pastilles (34 mmoles) et 10 ml d'eau distillée. On refroidit le mélange réactionnel à 5°C et on ajoute par fraction 5,60 g de D-L-Phénylalanine (34 mmoles). On agite pendant 15 min. en laissant la température remonter jusqu'à 20°C. On ajoute 0,3 g d'Aliquat® 336 (5% masse/masse de D,L-Phénylalanine) et on agite à nouveau à 500 tours/min durant 15 min. à 20°C. 6,30 g d'époxydodécane (34 mmoles) sont additionnés. Le brut réactionnel est chauffé à 100°C durant 5 heures 30 et refroidi à 20°C.

Le N-(2'-hydroxydodécyl) D,L-Phénylalaninate de potassium est obtenu avec un rendement de 96 %.

A titre comparatif, on a reproduit cet essai sans catalyseur de transfert de phase. Le produit fini est obtenu avec 77 % de rendement.

### Exemple 10

Dans un réacteur identique à celui de l'exemple 1, on introduit sous agitation 1,88 g de potasse en pastilles (34 mmoles) et 10 ml d'eau distillée. On refroidit le mélange réactionnel à 5°C et on ajoute par fraction 5,60 g de D,L-Phénylalanine (34 mmoles). On agite pendant 15 min en laissant la température remonter jusqu'à 20°C. On ajoute 0,3 g d'Aliquat® 336 (5% masse/masse de D,L-Phénylalanine) et on agite à nouveau à 500 tours/min durant 15 min. à 20°C. 9,93 g d'époxyhexadécane (34 mmoles en oxyde d'hexadécène calculé à partir de la valeur de l'indice d'oxirane du réactif de départ) sont additionnés. Le brut réactionnel est chauffé à 100°C durant 3 heures, (le milieu devient solide), puis refroidi à 20°C.

Le N-(2'-hydroxyhexadécyl) D,L-Phénylalaninate de potassium est obtenu avec un rendement de 96 %.

### Exemple 11

Dans un réacteur de 100 ml identique à celui de l'exemple 1, on introduit 3,20 g de N-méthylglucamine (16 mmoles), 10 ml d'eau distillée et 0,1 g d'Aliquat® 336 (3% masse/masse de N-méthylglucamine). On agite à 500 tours/min durant 15 min. à 20°C. 5,0 g d'époxyhexadécane (16 mmoles en oxyde d'hexadécène calculé à partir de la valeur de l'indice d'oxirane du réactif de départ) sont additionnés. Le brut réactionnel est chauffé à 100°C durant 5 heures et refroidi à 20°C.

Le N-(2'-hydroxyhexadécyl) méthylglucamine est obtenu avec un rendement de 95 %.

A titre comparatif, on a reproduit cet essai sans catalyseur de transfert de phase. Le produit fini est obtenu avec 79 % de rendement.

## Revendications

1. Procédé de préparation d'un composé hydroxylé d'amine secondaire ou tertiaire présentant une fonction hydroxyle en β de la fonction amine, à partir d'un composé amine, respectivement, primaire ou secondaire caractérisé en ce qu'on fait réagir ledit composé amine primaire ou secondaire avec un composé comportant une fonction époxy en présence(i)d'un catalyseur de transfert de phase et (ii) d'une base minérale puis, le cas échéant, on sépare le composé hydroxylé amine secondaire ou tertiaire formé, dudit catalyseur de transfert de phase.

2. Procédé selon la revendication 1 caractérisé en ce que le composé amine primaire ou secondaire comporte au moins un groupement oxygèné salifiable tel qu'un groupement -COO⁻, SO₃⁻, -SO₄⁻, -HPO₄⁻ ou -PO₃²⁻, ledit groupement étant sous forme libre ou salifiée.

3. Procédé selon l'une des revendications 1 et 2 caractérisé en ce que le composé amine primaire ou secondaire est un composé carboxyaminé comportant un ou deux groupes carboxyliques à l'état libre ou salifié.

4. Procédé selon la revendication 3 caractérisé en ce que le composé carboxyaminé est un acide aminé, un oligopeptide, un peptide, un acide amine N-acylé ou un composé carboxyaminé hétérocyclique comportant de 3 à 14 atomes de carbone et au moins un atome d'azote en tant qu'hétéroatome.

5. Procédé selon la revendication 4 caractérisé en ce que le composé carboxyaminé est un acide α aminé tel la sarcosine, la proline, la thréonine, l'histidine, l'asparagine, la valine, la phénylalanine, la leucine, l'isoleucine, la méthionine, le tryptophane, la lysine, l'alanine, l'acide glutamique, la glycine, la sérine, la tyrosine, la glutamine, la cystéine, la cystine, l'acide aspartique, l'arginine, l'hydroxyproline, l'hydroxylysine ou des mélanges de ces acides α aminés.

6. procédé selon la revendication 1 caractérisé en ce que l'amine primaire ou secondaire est la chitine ou un composé de formule (I)
R-NH - G (I)
dans laquelle R est l'hydrogène ou un radical alkyle en C₁ - ₂₀ et G est le reste d'un monosaccharide, d'un disaccharide, d'un polysaccharide ou d'un hexitol.

7. Procédé selon la revendication 6 caractérisé en ce que R est l'hydrogène ou un radical alkyle en C₁ - C₄, et G est le glucose ou le sorbitol.

8. Procédé selon l'une des revendications 1 à 7 caractérisé en ce que ledit composé comportant une fonction époxy est un composé de formule (II) 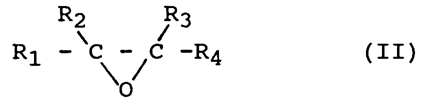 dans laquelle les substituants R₁, R₂, R₃ et R₄, identiques ou différents, représentent (i) l'hydrogène (ii), un groupe R₅ [(CH₂)ₛX]ₜ, R₅ étant un radical aliphatique, linéaire ou ramifié, saturé ou insaturé comportant de 1 à 30 atomes de carbone, un radical cycloaliphatique comportant de 3 à 12 atomes de carbone ou un radical aryle, R₅ étant ou non substitué par une à trois fonctions hydroxyles ou amines, ou (iii) un groupe R₆COOR₇, R₆ étant un radical alkylène en C₁-C₁₀ ou R₆ représente une liaison covalente et R₇ est H ou un radical aliphatique saturé ou insaturé, linéaire ou ramifié comportant de 1 à 30 atomes de carbone, X est l'oxygène ou le soufre, s est égal à 0 ou compris entre 1 et 4 et t est égal à 0 ou compris entre 1 et 20, sous réserve que l'un au moins de R₁, R₂, R₃, et R₄ est différent de l'hydrogène.

9. Procédé selon la revendication 8 caractérisé en ce que trois des substituants R₁, R₂, R₃ et R₄ représentent l'hdrogène, le quatrième substituant étant différent de l'hydrogène.

10. Procédé selon la revendication 9 caractérisé en ce que ledit substituant différent de l'hydrogène est un radical alkyle en C₁ - C₂₀, de préférence en C₈ - C₁₄.

11. Procédé selon l'une des revendications 1 à 10 caractérisé en ce que le catalyseur de transfert de phase est un composé d'ammonium quaternaire, de préférence un halogènure d'un composé d'ammonium quaternaire comme les bromures et chlorures de tétraalkylammonium ou de trialkylarylammonium, ou un dérivé organique de l'ion phosphonium.

12. Procédé selon l'une des revendications 1 à 10 caractérisé en ce que le composé amine primaire ou secondaire que l'on fait réagir avec le composé comportant une fonction époxy, est en solution dans l'eau.

13. Procédé selon l'une des revendications 1 à 12 caractérisé en ce que l'on fait réagir le composé amine primaire ou secondaire avec le composé comportant une fonction époxy en présence d'une base minérale.

14. Procédé selon la revendication 13 caractérisé en ce que la base minérale est LiOH, NaOH, KOH, Na₂CO₃, NaHCO₃, KHCO₃, K₂CO₃ ou NH₄OH.

15. Procédé selon l'une des revendications 1 à 4 caractérisé en ce que le rapport molaire entre le composé comportant une fonction époxy et le composé amine primaire ou secondaire est compris entre 0,5 et 10, et de préférence entre 0,8 et 1,20.

16. Procédé selon l'une des revendications 1 à 15 caractérisé en ce qu'on fait réagir le composé comportant une fonction époxy et le composé amine primaire ou secondaire à une température supérieure à 95°C, de préférence comprise entre 95°C et 160°C et plus préférentiellement comprise entre 95 et 125°C.

17. Composition caractérisée en ce qu'elle comporte au moins un composé hydroxylé d'amine secondaire ou tertiaire présentant une fonction hydroxylé en β de la fonction amine et un catalyseur de transfert de phase, ladite composition étant substantiellement exempte de solvant organique tels les alcools et les cétones.

18. Composition selon la revendication 17 caractérisée en ce que ledit composé hydroxylé et un composé de formule (III) : 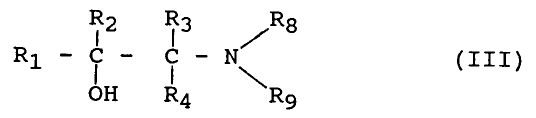 un composé de formule (IV) 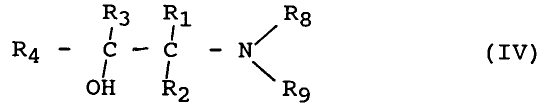 ou un mélange de composés de formule (III) et (IV),
dans lesquelles R₁, R₂, R₃ et R₄, identiques ou différents, sont tels que, définis dans la revendication 8, sous réserve que dans la formule (III), l'un de R₁ ou R₂ est différent de l'hydrogène et que dans la formule (IV), l'un de R₃ ou R₄ est différent de l'hydrogène,
R₈ et R_{9,}, identiques ou différents, représentent :
· l'une des significations de R₁
· un groupe R₁₀ - ZM, R₁₀ étant un radical aliphatique, linéaire ou ramifié, saturé ou insaturé ou un radical aryle, Z est un groupement oxygèné salifiable tel que -COO⁻, -SO₄⁻, -SO₃⁻, -HPO₄⁻ ou -PO₃²⁻ et M un contre-ion, tel NH₄ ou un cation d'un métal alcalin ou alcalino-terreux,
· un résidu d'un monosaccharide, d'un disaccharide, d'un polysaccharide ou d'un hexitol.
· un groupe 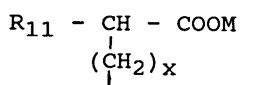 où R₁₁ est un reste d'un acide aminé, d'un oligopeptide ou d'un peptide, M a la signification indiquée ci-dessus et x est O ou un entier compris entre 1 et 5, ou
· R₈ et R₉ ensemble avec l'atome d'azote auxquels ils sont liés, forment un hétérocycle monocyclique ou polycyclique comportant de 3 à 14 atomes de carbone et, le cas échéant 1 à 3 hétéroatomes additionnels choisis parmi 0, S et N,
ledit hétérocycle étant substitué ou non par 1 ou 3 fonctions carboxyliques et/ou hydroxyles,
étant entendu que R₈ et R₉ ne représentent pas simultanément l'hydrogène.

19. Composition selon l'une des revendications 17 et 18, caractérisée en ce que ledit catalyseur de transfert de phase est un composé ammonium quaternaire, de préférence un halogénure d'un composé d'ammonium quaternaire.

20. Utilisation d'une composition selon l'une des revendications 17 à 19 comme agent tensio-actif notamment dans une composition cosmétique, pharmaceutique, hygiénique ou alimentaire.

21. Utilisation d'une composition selon l'une des revendications 17 et 19 en tant qu'agent détergent.
